# FASCICULE DE BREVET EUROPEEN

(11) **EP 2 011 888 B1**
(45) Date de publication et mention de la délivrance du brevet: **28.04.2010**
(21) Numéro de dépôt: 07025195.4
(22) Date de dépôt: 05.06.1990
(51) Int. Cl.: C12Q 1/70, C12Q 1/68, C12N 15/49

(54) **Séquences nucléotidiques issues du genome des rétrovirus du type HIV-1, HIV-2 et SIV, et leurs applications notamment pour l'amplification des genomes de ces rétrovirus et pour le diagnostic in vitro des infections dues à ces virus**
Nukleotidsequenzen des Genoms von Retroviren des Typs HIV-1, HIV-2 und SIV und deren Anwendungen, insbesondere zur Amplifikation von Genomen dieser Retroviren und für die In-Vitro-Diagnostik von durch diese Viren ausgelösten Infektionen
Nucleotide sequences resulting from the genome of the HIV-1, HIV-2 and SIV retroviruses, and their applications in particular for amplifying the genomes of these retroviruses and for in vitro diagnosis of infections caused by these viruses

(30) Priorité: 02.06.1989 FR 8907354; 20.09.1989 FR 8912371
(43) Date de publication de la demande: 07.01.2009
(62) Demande divisionnaire de: 05014676.0
(73) Titulaire: INSTITUT PASTEUR, 75724 Paris Cedex 15 (FR); INSTITUT NATIONAL DE LA SANTE ET DE LA RECHERCHE MEDICALE (INSERM), 75654 Paris Cédex 13 (FR)
(72) Inventeur: Moncany, Maurice, 75019 Paris (FR); Montagnier, Luc, Le Plessis-Robinsson (FR)
(74) Mandataire: Desaix, Anne

(56) Documents cités:
- EP-A- 0 229 701
- EP-A- 0 253 701
- EP-A- 0 269 520
- EP-A- 0 272 098
- OU C-H ET AL: "DNA amplification for direct detection of HIV-1 in DNA of peripheral blood mononuclear cells" SCIENCE, vol. 239, 15 janvier 1988 (1988-01-15), pages 295-297, XP002135449
- KWOK S ET AL: "IDENTIFICATION OF HUMAN IMMUNODEFICIENCY VIRUS SEQUENCES BY USING IN VITRO ENZYMATIC AMPLIFICATION AND OLIGOMER CLEAVAGE DETECTION" JOURNAL OF VIROLOGY,US,NEW YORK, US, vol. 61, no. 5, 1 mai 1987 (1987-05-01), pages 1690-1694, XP000616284 ISSN: 0022-538X
- RAYFIELD M ET AL: "Mixed human immunodeficiency virus (HIV) infection in an individual: Demonstration of both HIV type 1 and 2 proviral sequences by using polymerase chain reaction" THE JOURNAL OF INFECTIOUS MEDICINE, vol. 158, no. 9, 6 décembre 1988 (1988-12-06), pages 1170-76, XP002135450
- KEMP D J ET AL: "COLORIMETRIC DETECTION OF SPECIFIC DNA SEGMENTS AMPLIFIED BY POLYMERASE CHAIN REACTIONS" PROCEEDINGS OF THE NATIONAL ACADEMY OF SCIENCES OF USA, NATIONAL ACADEMY OF SCIENCE, WASHINGTON, DC, US, vol. 86, no. 7, 1 avril 1989 (1989-04-01), pages 2423-2427, XP000268576 ISSN: 0027-8424
- CHAKRABARTI ET AL: "Sequence of simian immunodeficiency virus from macaque and its relationship to other human and simian retroviruses" NATURE, vol. 328, August 1987 (1987-08), pages 543-547,

## Description

La présente invention est relative à des séquences oligonucléotidiques utilisables pour la mise en oeuvre de techniques d'amplification de séquences nucléiques spécifiques de rétrovirus d'immunodéficience humaine du type HIV ou de rétrovirus d'immunodéficience du singe du type SIV.

L'application de ces séquences à des méthodes de diagnostic in vitro chez l'homme de l'infection d'un individu par un rétrovirus du type HIV (actuellement HIV-1 et/ou HIV-2) est concernée en particulier.

L'isolement et la caractérisation de rétrovirus regroupés sous les désignations HIV-1 et HIV-2 ont été décrits dans les demandes de brevet européen EP 201540 et EP 239425 respectivement. Ces rétrovirus ont été isolés chez plusieurs malades présentant des symptômes d'une lymphadénopathie ou d'un Syndrome d'Immunodeficience Acquise (SIDA).

Les rétrovirus du type HIV-2 comme les rétrovirus du type HIV-1, se caractérisent par un tropisme pour les lymphocytes T4 humains et par un effet cytopathogène à l'égard de ces lymphocytes lorsqu'ils s'y multiplient, pour alors causer entre autres des polyadénopathies généralisées et persistantes, ou un SIDA.

Un autre rétrovirus, dénommé SIV-1, cette dénomination remplaçant la dénomination antérieurement connue STLV-III, a été isolé chez le singe macaque rhésus (M.D. DANIEL et al. Science, 228, 1201 (1985) N.L. LETWIN et al, Science, 230, 71 (1985) sous l'appellation "STLV-IIImac").

Un autre rétrovirus, désigné "STLV-IIIAGA", (ou SIV_{AGN}) a été isolé chez des singes verts sauvages. Mais contrairement aux virus présents chez le singe macaque rhésus, la présence de STLV-III_{AGN} ne semble pas induire une maladie du type SIDA chez le singe vert d'Afrique.

Pour la commodité du langage, ces virus ne seront plus désignés dans ce qui suit que par l'expression SIV (l'expression SIV est l'abréviation anglaise de "Simian Immunodeficency Virus" (Virus d'immunodéficience du singe) éventuellement suivie d'une abréviation désignant l'espèce de singe dont ils sont issus par exemple "MAC" pour le macaque" ou "AGM" pour le singe vert d'Afrique (abréviation de "African Green Monkey").

Une souche du rétrovirus SIV-IMac à été déposée à la C.N.C.M le 7 février 1986 sous le n° I-521.

La poursuite de l'étude des rétrovirus HIV-1 et HIV-2 a également conduit à l'obtention de séquences d'ADN complémentaires (ADNc) des ARN de leur génome. La séquence nucléotidique complète d'un ADNC d'un rétrovirus représentatif de la classe HIV-2 (HIV-2 ROD) a été déposée le 21/02/1986 à la C.N.C.M. sous le n° I-522, sous le nom de référence LAV-2 ROD.

De même, la séquence nucléotidique complète d'un ADNc d'un rétrovirus représentatif de la classe HIV-1 est décrite par WAIN HOBSON, SONIGO, COLE, DANOS et ALIZON dans CEll (janvier 1985).

Egalement pour la commodité du langage, les virus du type HIV-1 et HIV-2 seront parfois désignés dans ce qui suit par l'expression HIV.

Les méthodes de diagnostic in vitro des infections par des virus du type HIV-1 ou HIV-2 existant actuellement, font appel à la détection d'anticorps ANTI-HIV-1 ou anti-HIV-2 éventuellement présents dans un prélèvement biologique (biopsie) ou dans un fluide biologique, par exemple dans un sérum obtenu, à partir du patient à l'étude, par mise en contact de ce fluide biologique avec des extraits ou antigènes d'HIV-1 ou d'HIV-2, dans des conditions permettant la production d'une réaction immunologique éventuelle de ces ces extraits ou antigènes avec ces anticorps.

De telles méthodes de diagnostic risquent d'être faussement négatives, en particulier dans le cas d'une infection récente d'un individu par les virus du type HIV.

Les techniques d'amplification génique sont d'un appoint considérable pour la mise au point de méthodes de diagnostic in vitro particulièrement sensibles de maladies virales. Parmi ces techniques d'amplification génique, on peut citer la technique PCR (Polymérase Chain Reaction) telle que décrite dans les demandes de brevet européen EP200362 et EP229701, ou encore la technique dite "Qβreplicase" décrite dans Biotechnology, vol.6, page 1197 (octobre 1988) et celle procédant à l'aide d'une ARN polymérase (T7RNA polymérase) décrite dans la demande de brevet international n° WO89/01050. ces techniques permettent d'améliorer la sensibilité de détection des acides nucléiques des virus, et nécessitent l'utilisation d'amorces de synthèse spécifiques.

Pour la recherche des virus du type HIV, le choix des amorces est problématique. En effet, du fait de la grande variabilité des séquences de nucléotides du génome viral, une amorce conforme à la séquence connue d'un isolat donné d'un virus du type HIV peut faillir à l'amplification de certains variants viraux du type HIV. D'autre part, même si une amorce est choisie dans une région conservée du génome d'un virus HIV à un autre, son "bon fonctionnement" n'est pas pour autant assuré et peut donner lieu à de mauvais rendements d'amplification.

La présente invention fournit précisément des amorces oligonucléotidiques permettant, entre autres, l'amplification, notamment à des fins diagnostiques, du génome de tous virus du type HIV et SIV, avec des rendements considérés comme maximum dans l'état actuel de la technique et surtout évitant la présence de nombreuses bandes aspécifiques.

Les amorces de la présente invention sont à la fois spécifiques des virus du groupe HIV-1 et des virus des groupes HIV-2 et SIV, et sont insensibles aux variations du génome de ces virus.

La présente invention a pour objet des amorces oligonucléotidiques, d'environ 15 à 30 nucléotides, utilisables pour l'amplification génomique des virus du type HIV-1 et du type HIV-2 et SIV.

La demande décrit toute séquence nucléotidique caractérisée en ce que sa séquence :
- soit est choisie parmi celles qui sont contenues dans l'une des séquences nucléotidiques comprises dans les gènes gag, vpr et pol des virus HIV-1 Bru, HIV-1 Mal, HIV-1 Eli, HIV-2 ROD et SIV MAC, ou dans les gènes nef2, vif2 et vpx des virus HIV-2 ROD, et SIV MAC, ou dans les gènes env, nef1, vif1 et vpr des virus HIV-1 Bru, HIV-1 Mal, et HIV-1 Eli, et plus particulièrement parmi celles qui sont contenues dans les enchaînements nucléotidiques définis ci-après,
- soit (notamment pour les séquences les plus longues) contient l'une des séquences nucléotidiques susdites issues de HIV-1 Bru, ou HIV-1 Mal, ou HIV-1 Rayfield M et al. (Mixed human immunodeficiency virus (HIV) infection in an individual: Démonstration of both HIV type 1 and 2 proviral séquences by using polymerase chain reaction, The Journal Of Infectious Medicine, vol. 158, no. 9, 6 décembre 1988, pages 1170-76) décrit un couple d'amorces oligonucléotidiques SK100 et SK104 pour la mise en oeuvre d'une amplification génique du gène gag de virus du type HIV-1 et HIV-2, les amorces consistant chacune en une séquence spécifique du gène gag. Eli ou HIV-2 ROD ou SIVMac, ou contient une séquence nucléotidique complémentaire de l'une de ces dernières séquences, étant entendu que les nucléotides supplémentaires éventuels qui "débordent" la séquence nucléotidique du genre en question, du côté des extrémités 3' ou 5', coïncident de préférence avec ceux qui se trouvent placés en deçà des extrémités 5' ou 3' correspondant au sein même de la séquence complète des virus du type HIV-1, HIV-2 ou de SIV MAC, sus-mentionnés,
- soit, si cette séquence nucléotidique n'est pas identique à l'une des séquences nucléotidiques susdites, ou n'est pas complémentaire de l'une de ces séquences, est néanmoins susceptible de s'hybrider avec une séquence nucléotidique issue des virus HIV-1 Bru, HIV-1 Mal, HIV-1 Eli, et/ou avec une séquence nucléotidique issue du virus HIV-2 ROD ou SIV MAC sus-mentionnée. L'hybridation peut s'effectuer à une température de 60°C ± 1°C (de préférence 60°C ± 0,5°C), préconisée pour un optimum de rendement.

La numérotation des nucléotides mentionnés ci-dessous correspond à celle utilisée dans le manuel de référence "Human Retrovirus and AIDS-1989" édité par le "Los Alamos National Laboratory- New Mexico - USA". (Les séquences des virus HIV-1 Mal, HIV-1 Eli ont été décrites par MONTAGNIER, SONIGO, WAIN-HOBSON et ALIZON dans la demande de brevet européen n° 86.401380 du 23/06/86).

Les séquences sont synthétisées sur synthétiseur commercialisé par Applied Biosystems (méthode phosphoro-amidites, ou sur tout autre appareil utilisant une méthode semblable.

La demande concerne plus particulièrement les séquences oligonucléotidiques caractérisées par les enchaînements nucléotidiques suivants (représentés dans le sens 5' - 3'; les initiales "s" et "AS" indiquent si l'oligonucléotide est sens ou antisens, c'est-à-dire si l'oligonucléotide est orienté respectivement dans le sens 5'⊶ 3' ou dans le sens 3' ⊶ 5') :
1°) séquences communes aux génomes des virus HIV-1, HIV-2 et SIV (les séries de chiffres espacées d'un trait indiquent la position des nucléotides sur les génomes correspondant respectivement aux virus HIV-1 Bru, HIV-1 Mal, HIV-1 Eli, HIV-2 ROD et SIV) :
   séquences spécifiques du gène gag du génome des virus sus-mentionnés (gène codant pour un groupe d'antigènes spécifiques du nucléoïde de ces virus).
      Certaines variantes peuvent être apportées sur certaines positions des séquences nucléotidiques indiquées ci-dessous, sans que les propriétés d'hybridation de ces séquences nucléotidiques avec les gènes des virus du type HIV et/ou SIV soient affectées. Les séquences nucléotidiques comportant ces variantes sont représentées en-dessous des séquences nucléotidiques initiales dont elles dérivent par remplacement d'une ou plusieurs bases. Les bases modifiées par rapport à celles des séquences nucléotidiques initiales sont indiquées en toute lettre à la verticale des positions correspondant aux bases qui ont été remplacées dans ces séquences initiales ; tandis que les bases des séquences initiales qui n'ont pas été remplacées dans les séquences comportant ces variantes sont indiquées à l'aide de pointillés.
      La synthèse des amorces se fait en utilisant toutes les variantes simultanément. C'est le mélange de toutes les variantes pour une séquence donnée qui est utilisé dans les tests. 
MMy1 :   TGG CGC CCG AAC AGG GAC
         ... ... .T. ... ... ...
         S, 636-653, 635-652, 636-653, 859-876, 834-851
 
MMy2 :    GGC CAG GGG GAA AGA AAA A
          ... .C. .C. ... ... ... .
          ... ... .A. ... ... ... .
          S,854-872,864-888,848-872,1160-1184, 1124-1148
 
MMy3 :    TGC CCA TAC AAA ATG TTT TA
          ... ... C.. T.T ... ... ..
AS,900-881,916-897,900-881,1212-1193,1176-1157
 
MMy4 : TGC ATG GCT GCT TGA TG
... ..A ... ..C ..G ..
AS,1385-1369,1419-1403,1385-1369,1703-1687,1667-1651
 
MMy4B : CTT TGC ATG GCT GCT TGA TG
..C ... ..A ... ..C ..G ..
AS, 1388-1369, 1421-1403, 1388-1369, 1706-1687,
1670-1651,
 
MMy4Bbis : CAT CAA GCA GCC ATG CAA AG
..C ..G ... ..T ... ..G ..
S, 1369-1388, 1403-1421, 1369-1388,
1687-1706, 1651-1670,
 
MMy28 : AGG GCT GTT GGA AAT GTG G
... ... ... ... ..G ... .
S, 2021-2039, 2055-2073, 2024-2042, 2329-2349,
2299-2318,
 
MMy28bis : CCA CAT TTC CAG CAT CCC T
... ... ... ... ..G ... .
... ... ... ... ..C ... .
AS, 2039-2021, 2073-2055, 2042-2024, 2349-2329,
2318-2299
 séquences spécifiques du gène vpr 
MMy18 : GAT AGA TGG AAC AAG CCC CAG
S, 5590-5610, 5585-5605, 5554-5574, 6233-6296,
6147-6170,
 
MMy19 : TCC ATT TCT TGC TCT CCT CTG T
AS, 5870-5849, 5865-5844, 5834-5813.
6551-6531, 6454-6431,
 séquences spécifiques du gène pol : 
MMy29 : TAA AGC CAG GAA TGG ATG GCC CAA
... ... ... ... ... ... .A. ...
S, 2620-2643, 2615-2638, 2584-2607, 2971-2994,
2887-3010
 
MMy29bis : TTG GGC CAT CCA TTC CTG GCT TTA
... .T. ... ... ... ... ... ...
AS, 2643-2620, 2638-2615, 2607-2584, 2994-2971,
3010-2887,
 
MMy30 : TGG ACT GTC AAT GAC ATA CAG AA
... ... ... ... ..T ... ... ..
S, 3339-3361, 3334-3356, 3303-3325, 3690-3712,
3606-3628,
 
MMy30bis : TTC TGT ATG TCA TTG ACA GTC CA
... ... ... ... ... ..T ... ..
AS, 3361-3339, 3356-3334, 3325-3303, 3712-3690,
3628-3606,
 
MMy31 : CAT GGG TAC CAG CAC ACA AAG G
S, 4186-4207, 4181-4202, 4150-4171, 4534-4555,
4450-4471,
 
MMy31bis : CCT TTG TGT GCT GGT ACC CAT G
AS, 4207-4186, 4202-4181, 4171-4150, 4555-4534,
4471-4450,
 
MMy32 : TGG AAA GGT GAA GGG GCA GT
... ... ... ... ..A ... ..
S, 4992-5011, 4987-5006, 4956-4975, 5340-5359,
5256-5275,
 
MMy32bis : ACT GCC CCT TCA CCT TTC CA
... ... ... ..T ... ... ..
... ... ... ..C ... ... ..
AS, 5011-4992, 5006-4987, 4975-4956, 5359-5340,
5275-5256
2°) séquences communes aux génomes des virus HIV-2 et SIV (les séries de chiffres espacées d'un trait indiquent la position des nucléotides sur les génomes correspondant respectivement aux virus HIV-2 ROD, et SIV-MAC).
   - séquences spécifiques du gène nef2
      (codant pour un facteur négatif de 27 kD) 
MMy12 : AGA GAC TCT TGC GGG CGC GTG
S, 9165-9185, 9139-9159,
 
MMy13 : ATA TAC TTA GAA AAG GAA GAA GG
S, 9542-9564, 9516-9538,
 
MMyl3bis : CCT TCT TCC TTT TCT AAG TAT AT
AS, 9564-9542, 9538-9516,
 
MMy14 : AGC TGA GAC AGC AGG GAC TTT CCA
AS, 9956-9933, 9893-9870,
   - séquences spécifiques du gène vif2 (codant pour
      un facteur d'infectiosité de 23 kD) 
MMy20 : TAT GGA GGA GGA AAA GAG ATG GAT AGT
S, 5424-5450, 5340-5366,
 
MMy21 : TAG CAC TTA TTT CCC TTG CTT T
S, 5754-5775, 5670-5691,
 
MMy21bis : AAA GCA AGG GAA ATA AGT GCT A
AS, 5775-5754, 5691-5670,
 
MMy22 : CCC TTG TTC ATC ATG CCA GTA T
AS, 6082-6061, 5995-5974,
   - séquences spécifiques du gène vpx (codant pour
      une protéine de 12 kD) 
MMy23 : ATG TCA GAT CCC AGG GAG A
S, 5900-5918, 5813-5831,
 
MMy24 : CCT GGA GGG GGA GGA GGA GGA
AS, 6228-6208, 6141-6121,
3°) Séquences communes aux génomes des virus HIV-1 Bru, HIV-1 Mal, et HIV-1 Eli (les séries de chiffres espacées d'un trait indiquent la position des nucléotides sur les génomes correspondant respectivement aux virus HIV-1 Bru, HIV-1 MAl et HIV-1 Eli).
   - séquences spécifiques du gène env (codant pour les protéines d'enveloppe)

MMy5 : CCA ATT CCC ATA CAT TAT TGT GCC CC
5,6905-6930,6903-6928,6860-6885

MMy5bis : GGG GCA CAA TAA TGT ATG GGA ATT GG
AS, 6930-6905, 6928-6903, 6885-6860,

MMy6 : AAT GGC AGT CTA GCA GAA GAA GA
S,7055-7077,7053-7075,7010-7032

MMy7 : ATC CTC AGG AGG GGA CCC AGA AAT T
S, 7360-7384, 7349-7373,7306-7330

MMy7bis : AAT TTC TGG GTC CCC TCC TGA GGA T
AS, 7384-7360,7373-7349,7330-7306

MMy8 : GTG CTT CCT GCT GCT CCC AAG AAC CC
AS, 7857-7832,7846-7821,7800-7775

MMy8bis : GGG TTC TTG GGA GCA GCA GGA AGC AC
S, 7832-7857, 7821-7846, 7775-7800,

MMy9 : ATG GGT GGC AAG TGG TCA AAA AGT AG
... ... ... ..A ... ... ... ... ..
S,8844-8869, 8836-8861, 8787-8812,

MMy9bis : CTA CTT TTT GAC CAC TTG CCA CCC AT
AS, 8869-8844, 8861-8836, 8812-8787,

MMy78 : TAT TAA CAA GAG ATG GTG G
S, 7629-7647, 7612-7630, 7572-7590,

MMy89 : CCA GCA AGA AAA GAA TGA A
S, 8224-8242, 8213-8231, 8167-8185,

MMy89bis : TTC ATT CTT TTC TTG CTG G
AS, 8242-8224, 8231-8213, 8185-8167,
. séquences spécifiques du gène nef1

MMy10 : AAA AGA AAA GGG GGC ACT GGA
S, 9116-9136, 9117-9137, 9062-9082,

MMy10bis : TCC AGT CCC CCC TTT TCT TTT
AS,9136-9116, 9137-9117, 9082-9062,

MMy11 : AAA GTC CCC AGC GGA AAG TCC C
AS,9503-9483, 9505-9484, 9449-9428,
. séquences spécifiques du gène vif 1

MMy15 : GAT TAT GGA AAA CAG ATG GCA GGT GAT
S, 5073-5099, 5068-5094, 5037-5063,

MMy16 : GCA GAC CAA CTA ATT CAT CTG TA
S, 5383-5405, 5378-5400, 5347-5369,

MMy16bis : TAC AGA TGA ATT AGT TGG TCT GC
AS, 5405-5383, 5400-5378, 5369-5347,

MMy17 : CTT AAG CTC CTC TAA AAG CTC TA
AS, 5675-5653, 5670-5648, 5639-5617,
. séquences spécifiques du gène vpu

MMy25 : GTA AGT AGT ACA TGT AAT GCA ACC T
S, 6081-6105, 6076-6100, 6045-6069,

MMy26 : AGC AGA AGA CAG TGG CCA TGA GAG
S, 6240-6263, 6238-6261, 6207-6230,

MMy27 : ACT ACA GAT CAT CAA TAT CCC AA
AS, 6343-6321, 6338-6316, 6307-6285,

La demande a également pour objet les séquences (ou amorces) possédant une structure nucléotidique complémentaire de celles des amorces définies ci-dessus.

Elle concerne également les séquences nucléotidiques présentant certaines mutations par rapport a celles définies ci-dessus sans que les propriétés d'hybridation, telles que définies ci-dessus, de ces séquences soient modifiées. Le pourcentage de nucléotides différents de ceux constituant les séquences décrites ci-dessus, sans pour autant affecter les propriétés d'hybridation des séquences de l'invention, peut atteindre 40 %.

D'une manière générale, dans le cas d'une amorce (primer) sens (S), un plus grand nombre de mutations seront tolérées du côté 5' que du côté 3' de l'amorce, le côté 3' devant s'hybrider parfaitement avec un brin déterminé d'une séquence nucléique pour permettre l'amplification de cette séquence. Dans le cas d'une amorce anti-sens(AS), la tolérance est permise du côté 3'.

La demande décrit également les amorces telles que définies ci-dessus et comportant une conservation d'au moins 5 bases de chaque côté, la partie médiane comportant des modifications, sans que le propriétés d'hybridation ci-dessus soient modifiées.

Une des caractéristiques des amorces oligonucléotidiques décrites est de donner une bande d'amplification nette, dépourvue généralement de bandes aspécifiques lorsque les indications techniques d'utilisation décrites dans la présente invention sont mises en oeuvre. Ce fait est du à la longueur des amorces pouvant atteindre 27 bases ce qui accroît la spécificité d'hybridation, ainsi qu'aux conditions d'utilisation drastiques qui permettent d'éliminer les associations parasites. La spécificité pour chaque type de virus est fonction, outre du pourcentage d'homologie avec la matrice de référence, de la longueur des amorces, qui peuvent atteindre, pour un rendement acceptable, jusqu'à 40 bases.

La demande vise également des amorces telles que décrites ci-dessus liées au niveau de leur extrémité 5' à un promoteur pour la mise en oeuvre d'une méthode d'amplification génomique par synthèse de multiple copies d'ADN ou d'ARN telle que décrite dans la demande de brevet européenne EP 310229.

La demande a notamment pour objet l'utilisation des amorces décrites ci-dessus pour la mise en oeuvre d'un procédé d'amplification génique de séquences nucléiques de virus du type HIV-1 et/ou HIV-2, et/ou SIV, ce procédé étant applicable au diagnostic in vitro de l'infection potentielle d'un individu par un virus du type HIV-1 et/ou HIV-2 ou d'un animal par au moins l'un des trois virus (HIV-1, HIV-2, SIV).

Cette méthode de diagnostic in vitro est réalisée à partir d'un échantillon biologique (par exemple un fluide biologique tel que le sérum, les lymphocytes du sang circulant) obtenu à partir d'un patient à l'étude, et comprend principalement les étapes suivantes :
- une étape d'extraction de l'acide nucléique à détecter appartenant au génome du virus du type HIV-1 et/ou HIV-2 et/ou SIV éventuellement présent dans l'échantillon biologique sus-mentionné, et,le cas échéant, une étape de traitement à l'aide d'une transcriptase inverse dudit acide nucléique si ce dernier est sous forme d'ARN afin d'obtenir un acide nucléique double brin (cette dernière étape étant encore désignée ci-dessous par étape de rétro-transcription de l'ARN viral),
- un cycle comprenant les étapes suivantes :
   - dénaturation de l'acide nucléique double brin à détecter , ce qui conduit à la formation d'un acide nucléique simple brin,
   - hybridation de chacun des brins d'acide nucléique, obtenus lors de l'étape de dénaturation précédente, avec au moins une amorce selon l'invention, par mise en contact des brins sus-mentionnés avec au moins un couple d'amorces selon l'invention dans les conditions d'hybridation définies ci-dessous,
   - formation à partir des amorces des ADN complémentaires aux brins sur lesquels elles sont hybridées en présence d'un agent de polymérisation (ADN polymérase) et de quatre nucléosides triphosphate (dNTP) différents, ce qui conduit à la formation d'un plus grand nombre d'acides nucléiques double brin à détecter qu'à l'étape de dénaturation précédente, ce cycle étant répété un nombre de fois déterminé pour obtenir ladite séquence nucléique à détecter éventuellement présente dans l'échantillon biologique dans une proportion suffisante pour permettre sa détection,
- une étape de détection de la présence éventuelle de l'acide nucléique appartenant au génome du virus du type HIV-1 et/ou HIV-2 et/ou SIV dans l'échantillon biologique.

L'étape d'hybridation décrite ci-dessus est avantageusement réalisée à 60°C pendant 1 minute 30 secondes dans le tampon "10 X buffer" dont la composition (en concentration finale d'utilisation) est indiquée ci-dessous.

La méthode de diagnostic in vitro peut être réalisée soit à partir de l'ARN viral, soit à partir de l'ADN complémentaire, épisomial ou intégré.

En effet, les génomes des virus HIV et SIV se présentent sous forme d'ARN ou d'ADN en fonction de la localisation du virus dans l'organisme.

Lorsque le virus est situé à l'intérieur des cellules de l'organisme, notamment à l'intérieur des cellules sanguines, son ARN est recopié en ADN par une transcriptase inverse. En revanche, le génome des virus du type HIV en milieu extracellulaire, notamment dans le sang, demeure sous forme d'ARN.

L'étape d'extraction de l'ADN viral contenu dans les cellules de l'échantillon biologique préconisée par les inventeurs - outre la méthode classique au phénol chloroforme - présente les étapes suivantes : 1.
- suspension du culot cellulaire dans 0,5 ml d'eau pyrolisée dans un Potter gros piston,
- broyage des cellules dit par "aller et retour",
- adjonction Triton X100 pour 1 concentration finale de 0,1 %,
- dénaturation à la chaleur durant 15 à 25 minutes à 100°C,
- centrifugation courte pour n'éliminer que les débris cellulaires,
- précipitation de l'ADN durant la nuit à -20°C par adjonction de 2,5 volumes d'éthanol absolu et de 10 % du volume final d'acétate de sodium 3 Molaires. L'ADN est ensuite récupéré puis resuspendu dans l'eau pyrolysée après avoir été lavé 2 fois par de l'éthanol à 70°. Il est à noter que cette méthode permet la précipitation conjointe des ADN et des ARN, ce qui autorise la détection du message génomique des virus de types HIV ou SIV par utilisation de la méthode dite "PCR directe ADN" ou par celle dite de "PCR-ARN".

L'étape d'extraction de l'ARN viral est généralement effectuée de manière classique connue de l'homme de l'Art.

Après extraction de l'ARN, une étape supplémentaire de transformation de l'ARN, monobrin en ADN double brin est nécessaire à effectuer lorsque le diagnostic in vitro est réalisé à partir d'échantillons biologiques contenant les virus du type HIV-1 et/ou HIV-2 et/ou SIV dont les génomes sont sous forme d'ARN.

Cette transformation de l'ARN en ADN est réalisée par traitement de l'ARN obtenu après extraction de l'échantillon biologique, notamment du sérum, dans un milieu approprié à l'aide d'une transcriptase inverse. Est plus particulièrement décrite une méthode de diagnostic in vitro telle que définie ci-dessus, dans laquelle l'étape de rétro-transcription de l'ARN viral est réalisée de la manière suivante :
- 10 µg d'ARN extrait resuspendu dans de l'eau est mis en présence du couple d'amorces à la concentration de 40 µM chacun, dans un volume final de 40 µl. L'ensemble est dénaturé à 100°C durant 10 minutes puis plongé dans de l'eau glacée,
- l'on rajoute 10 µl du mélange suivant : 5 µl du tampon "10 X buffer" décrit ci-dessous + 1 unité de reverse-transcriptase (d'AMV (Avian Myeloblastosis virus) ou de MuMLV (Moloney Leukemia Virus)) + 1 unité de Taq-polymérase + 1 µl du mélange des 4 dNTP à 25 mM chacun + de l'eau Q.S.P. 10 µl. Le volume final est donc de 50 µl.
   Cette réaction s'effectue en deux étapes :
   - a) 1ère étape : fabrication de l'ADNc par action de la réverse transcriptase à 42°C durant 13 minutes,
   - b) 2ème étape : amplification génique classique: on chauffe à 95°C durant 3 minutes pour détruire la réverse-transcriptase et permettre l'étape de déshybridation/hybridation, puis on démarre le cycle décrit précédemment pour l'amplification génique.

   Est plus particulièrement décrite une méthode de diagnostic in vitro telle que décrite ci-dessus, dans laquelle l'étape de dénaturation est réalisée en présence du (ou des) couple(s) d'amorces (ou de primers) décrits. En effet, comme il a été précisé ci-dessus, une des caractéristiques des oligonucléotides (ou primers) décrits est de donner une bande d'amplification nette, dépourvue généralement de bandes aspécifiques, lorsqu'ils sont utilisés dans les conditions qui suivent :
- hybridation ; les primers (µl d'une solution à 40 µmolaire (40 µM) de chaque primer) sont mis en présence de l'ADN-matrice (100 à 300 ng) pour la première étape de dénaturation-réascociation : on chauffe, durant 10 minutes a 100°C puis on plonge les tubes contenant ce mélange d'ADN-matrice et de primers dans de l'eau contenant de la glace afin d'augmenter le taux de réassociation ADN-matrice/primers. Les primers doivent être utilisés à une concentration finale dans l'étape d'amplification qui suit de 0,8 µM chaque.
- amplification : on ajoute au milieu précédent les 4 A dNTP chacun étant utilisé à 0,5 µmolaire en solution finale (50 µl), et une unité de Taq-polymérase pour un milieu réactionnel de 50 µl ; cette étape est réalisée dans un tampon d'amplification de la présente invention, généralement désigné sous le nom de "10 X buffer" dont la composition (lorsqu'il est dilué au 1/10°) est la suivante : Tris-HCl, pH 8,9 : 50 mM ; (NH₆)₂ SO₆ : 15 mM ; MgCl2 : 5 mH ; β-mercapto-éthanol : 10 mM ; gélatine : 0,25 mg/ml. On additionne 5 µl de ce tampon et de l'eau q.s.p. 50 µl au milieu précédent.

Les cycles d'amplification sont réalisés de la manière suivante : 30 à 40 cycles composés de :
- 94°C durant 10 secondes (dénaturation),
- 60°C durant 1 minute 30 (hybridation),
- 78°C durant 1 minute 30 (élongation).

Le tout sera suivi par un cycle unique à 78°C durant 15 minutes.

La précision des températures indiquées à ± 0,3°C près, ainsi que leur stabilité durant les différents cycles, représentent des conditions essentielles pour l'obtention des rendements maximum ainsi que l'absence de bandes aspécifiques.

La concentration optimale d'ADN est de 100 à 300 ng pour de l'ADN génomique extrait de cellules (de patients ou en culture, de mammifères ou autres).

Il va de soi que les conditions qui précèdent représentent des conditions optimales pour un milieu réactionnel final de 50 µl, et que ces conditions peuvent être modifiées en fonction du volume final du milieu réactionnel.

L'utilisation de plusieurs couples d'amorces différents (ou coktails de couples) permet soit la détection croisée de plusieurs types de virus du type HIV et/ou SIV, soit la détection simultanée de plusieurs gènes d'un même virus du type HIV et/ou SIV.

A titre d'exemple de couples d'amorces préférés, on peut citer les couples d'amorces suivants :
- MMyl-MMy4, MMy2-MMy4, MMyl-MMy3, MMy18-MMy19, Mmy4bis-MMy28bis, MMy28-MMy29bis, MMy29-MMy30bis, MMy31-MMy32bis, notamment pour le diagnostic in vitro de l'infection d'un individu par HIV-1 et/ou HIV-2
- MMy5-MMy8, MMy6-MMy8, MMy7-MMy8, MMy5-MMy7bis, MMy6-MMy7bis, MMy9-MMy11, MM10-My11, MMy9-MMy10bis, MMy26-MMy5bis, MMy8bis-MM9bis, MMy8bis-MMy89, MMy89bis-MMy9bis, MMy15-MMy17, MMy15-MMy16bis, MMy16-MMy17, MM25-MMy27, MMy26-MMy27, notamment pour le diagnostic in vitro de l'infection d'un individu par HIV-1,
- MMy20-MMy22, MMy20-MMy21bis, MMy21-MMy22, MMy23-MMy24, MMy12-MMy14, MMy12-MMy13bis, pour le diagnostic in vitro de l'infection d'un individu par HIV-2.

L'agent de polymérisation utilisé dans l'étape d'élongation du cycle est une ADN polymérase thermostable, notamment la Taq polymérase, l'amplifiose de la firme Appligène ou toute ADN-polymérase thermostable pouvant être commercialisée.

D'une manière générale, le cycle de la méthode de diagnostic in vitro est répété entre 30 et 40 fois.

La méthode de diagnostic in vitro permet également, en fonction des couples d'amorces nucléotidiques utilisés, de détecter sélectivement les gènes des virus du type HIV et/ou SIV présents dans l'échantillon biologique.

Les couples d'amorces utilisables, à titre d'exemples, pour la méthode de diagnostic gène par gène sus-mentionnée, sont les suivants :
- MMy1-MMy4, MMy2-MMy4, MMyl-MMy3, MMy4bis-MMy28bis pour le gène gag,
- MMy18-MMy19 pour le gène vpr,
- MMy5-MMy8, MMy6-MMy8, MMy7-MMy8, MMy5-MMy7bis, MMy6-MMy7bis, MMy26-MMy5bis, MMy8bis-MMy9bis, MMy8bis-MMy89, MMy89bis-MMy9bis pour le gène env,
- MMya-MMy11, MMy9-MMy10bis, MMy10-MMy11 pour le gène nef1,
- MMy15-MMy17, MMy15-MMy16bis, MMy16-MMy17, pour le gène vif1,
- MMy20-MMy22, MMy20-MMy21bis, MMy21-MMy22 pour vif 2,
- MMy23-MMy24 pour vpx,
- MMy12-MMy14, MMy12-MMy13bis, MMy13-MMy14 pour nef2,
- MMy25-MMy27, MMy26-MMy27 pour le gène vpu,
- MMy28-MMy29bis, MMy29-MMy30bis, MMy30-MMy31bis, MM31-MMy32bis pour le gène pol.

Toutefois, les combinaisons entre primers "S" et "AS" décrites ci-dessus ne sont par limitatives et peuvent être variées selon le désir de l'utilisateur.

Les tailles des fragments nucléotidiques synthétisés à l'aide des couples d'amorces sus-mentionnés à titre d'exemples, sont indiquées sur les tableaux I à XI suivants :
(les chiffres indiqués dans les tableaux ci-dessous représentent le nombre de nucléotides des fragments synthétisés, et les "tirets" indiquent que les couples d'amorces testés ne permettent pas de caractériser les souches virales correspondantes).

**Tableau I**

| | | | | | | | | |
|---|---|---|---|---|---|---|---|---|
| | gag | | | : | gag | | | : |
| | :MMyl-MMy3:MMy1-MMy4:MMy2-MMy4:MMy4bis-MMy28bis: | | | | | | | |
| HIV1-BRU: | 265 | : | 750 | : | 532 | : | 671 | : |
| HIV1-ELI: | 282 | : | 785 | : | 556 | : | 671 | : |
| HIV1-ELI: | 265 | : | 750 | : | 538 | : | 674 | : |
| HIV2-ROD: | 354 | : | 845 | : | 544 | : | 663 | : |
| SIV : | 343 | : | 844 | : | 544 | : | 668 | : |

**Tableau II**

| | | | | | | | | |
|---|---|---|---|---|---|---|---|---|
| | env | | : | | env | | | : |
| :MMy5-MMy7bis:MMy5-MMy8:MMy6-MMy7bis:MMy6-MMy8 : | | | | | | | | |
| HIV1-BRU: | 480 | : | 953 | : | 330 | : | 803 | : |
| HIV1-MAL: | 471 | : | 944 | : | 321 | : | 794 | : |
| RIV1-ELX: | 471 | : | 941 | : | 321 | : | 791 | : |
| HIV2-ROD: | - | : | - | : | - | : | - | : |
| SIV : | - | : | - | : | - | : | - | : |

**Tableau III**

| | | | | | | |
|---|---|---|---|---|---|---|
| | env | | | : | env | : |
| :MMy7-MMy8:MMy26-MMy5bis:HMy8bis-MMy9bis | | | | | | : |
| HIV1-BRU: | 498 | : | 691 | : | 1038 | : |
| HIV1-MAL: | 198 | : | 691 | : | 1041 | : |
| HIV1-ELI: | 195 | : | 679 | : | 1038 | : |
| HIV2-ROD: | - | : | - | : | - | : |
| SIV : | - | : | - | : | - | : |

**Tableau IV**

| | | | | |
|---|---|---|---|---|
| | env | : | env | : |
| | :MMy8Bis-MMy89 | : | MMy89bis-MMy9bis | : |
| HIVl-BRU: | 411 | : | 646 | : |
| HIV1-MAL: | 411 | : | 649 | : |
| HIV1-ELI: | 411 | : | 646 | : |
| HIV2-ROD: | - | : | - | : |
| SIV : | - | : | - | : |

**Tableau V**

| | | | | | | |
|---|---|---|---|---|---|---|
| | nef1 | : | nef1 | | | : |
| | :MMy9-MMy10bis | : | MMy9-MMy11 | : | MMy10-MMy11 | : |
| HIV1-BRU: | 293 | : | 660 | : | 388 | : |
| HIV1-MAL: | 302 | : | 660 | : | 388 | : |
| HIV1-ELI: | 296 | : | 663 | : | 388 | : |
| HIV2-ROD: | - | : | - | : | - | : |
| SIV : | - | : | - | : | - | : |

**Tableau VI**

| | | | | | | |
|---|---|---|---|---|---|---|
| | nef2 | | | : | nef2 | : |
| | MMy12-MMy13bis:MMy12-MM14:MMy13-MMy14: | | | | | |
| HIV1-BRU: | - | : | - | : | - | : |
| HIV1-HAL: | - | : | - | : | - | : |
| HIV1-ELI: | - | : | - | : | - | : |
| HIV2-ROD: | 400 | : | | : | 415 | : |
| SIV : | 400 | : | 755 | : | 378 | : |

**Tableau VII**

| | | | | | | |
|---|---|---|---|---|---|---|
| | vif1 | | | | : | vif1 |
| | :MMy15-MMy16bis:MMy15-MMy17:MMy16-MMy17 | | | | | : |
| HIV1-BRU: | 333 | : | 603 | : | 293 | : |
| HIVI-MAL: | 333 | : | 603 | : | 293 | : |
| HIV1-ELI: | 333 | : | 603 | : | 293 | : |
| HIV2-ROD: | - | : | - | : | - | : |
| SIV | - | : | - | : | - | : |

**Tableau VIII**

| | | | | | | |
|---|---|---|---|---|---|---|
| vpr | | : | vif2 | | | : |
| :MMy18-MMy19 | | : | MMy20-MMy21bis | : | MMy20-MMy22 | : |
| HIV1-BRU: | 281 | : | - | : | - | : |
| HIV1-MAL: | 281 | : | - | : | - | : |
| HIV1-BLI: | 281 | : | - | : | - | : |
| HIV2-ROD: | 319 | : | 352 | : | 659 | : |
| SIV : | 308 | : | 352 | : | 656 | : |

**Tableau IX**

| | | | | |
|---|---|---|---|---|
| | vif2 | : | vpx | : |
| : | MMy21-MMy22 | : | MMy23-MMy24 | : |
| HIV1-BRU: | - | : | - | : |
| HIV1-MAL: | - | : | - | : |
| HIV1-ELI: | - | : | - | : |
| HIV2-ROD: | 329 | : | 329 | : |
| : SIV : | 326 | : | 329 | : |

**Tableau X**

| | | | | | | |
|---|---|---|---|---|---|---|
| | vpu | | | : | pol | : |
| | :MMy25-MMy27 | : | MMy26-MMy27 | : | MMy28-MMy29bis | : |
| HIV1-BRU: | 263 | : | 104 | : | 623 | : |
| HIV1<MAL: | 263 | : | 101 | : | 584 | : |
| HIV1-ELI: | 263 | : | 101 | : | 584 | : |
| HIV2-ROD: | - | : | - | : | 666 | : |
| SIV : | - | : | - | : | 712 | : |

**Tableau XI**

| | | | | | | |
|---|---|---|---|---|---|---|
| pol | | | | : | pol | : |
| | :MMy29-MMy30bis:MMy30-MMy31bis:MMy31-MMy32bis | | | | | : |
| HIV1-BRU: | 742 | : | 869 | : | 826 | : |
| HIVI-MAL: | 742 | : | 869 | : | 826 | : |
| HIV1-ELI: | 742 | : | 869 | : | 826 | : |
| HIV2-ROD: | 742 | : | 866 | : | 826 | : |
| SIV : | 742 | : | 866 | : | 826 | : |

Il est à noter que grâce à leur disposition sur le génome, les primers servant à l'amplification peuvent être combinés de telle façon qu'ils peuvent être utilisés comme sonde, soit après marquage au ³²P par kination, soit pour l'utilisation dans la technique des sondes froides pour vérifier la spécificité de la bande d'amplification observée lors d'une analyse par "Southern transfert". En plus de la combinaison classique des amorces pour qu'un troisième oligonucléotide puisse servir de sonde interne spécifique, il est à noter le cas particulier des gènes vif1/vpr et vif2/vpx dû au chevauchement de ces gènes, ce qui permet une détection croisée. En outre, lors d'une analyse par séquençage de l'ADN amplifié, ces oligonucléotides peuvent être utilisés comme amorce spécifique pour la DNA-polymérase permettant un double séquençage dans chaque sens, donc une double lecture des séquences, levant ainsi des ambiguïtés éventuelles d'interprétation.

Sont également décrites les amorces, telles que définies ci-dessus, marquées, notamment de manière radioactive ou enzymatique, ainsi que leur utilisation en tant que sondes nucléotidiques, notamment dans le cadre de la méthode de diagnostic in vitro telle que décrite ci-dessus. Sont également décrits des oligonucléotides tels que décrits ci-dessus et comportant des sucres en conformation a. De tels oligonucléotides présentent la caractéristique d'inverser le sens de la double hélice formée avec la matrice (brin du génome du virus), cette double hélice passant ainsi de l'état "S" à l'état "AS".

Sont concernés aussi les oligonucléotides décrits ci-dessus dont certains nucléotides sont méthylés et/ou comportent un ou plusieurs atomes de soufre notamment sur les adénines. De tels oligonucléotides présentent la caractéristique d'augmenter la stabilité de la double hélice, et par conséquent de mieux s'hybrider avec le brin d'ADN à amplifier.

Sont visés également les oligonucléotides tels que décrits ci-dessus et se présentant sous la forme dite "de bases modifiées" comportant des nucléotides sur lesquels sont greffés de façon covalente des agents chromophores (molécules aromatiques planes telles que l'acridine orange), notamment selon la méthode décrite dans l'article de C. Hélène paru dans "la Vie des Sciences", compte-rendus, série générale, tome 4, n"1, p. 17-37. De tels oligonucléotides présentent la caractéristique d'être facilement détectables, notamment par fluorescence.

Les oligonucléotides décrits sont également utilisables pour la mise en oeuvre d'une méthode de diagnostic in vitro de l'infection de singes (macaque, singe de mangabeys ou singe vert) par le virus du type SIV, cette méthode reprenant les principales caractéristiques de celle décrite ci-dessus.

Sont également décrits des kits de diagnostic pour la mise en oeuvre des méthodes de diagnostic in vitro sus-mentionnées. A titre d'exemple, un kit de diagnostic comprend :
- au moins un couple d'amorces oligonucléotidiques Selon la demande, chaque couple comprenant une amorce s'hybridant à l'un des brins de la séquence d'acide nucléique à détecter, et une amorce s'hybridant avec le brin complémentaire de ce dernier dans les conditions définies ci-dessus,
- des réactifs appropriés à la mise en oeuvre du cycle d'opérations d'amplification, notamment de l'ADN polymérase, et quatre nucléotides triphosphate différents, et le milieu réactionnel dénommé "10 X buffer" décrit ci-dessus.
- une (ou plusieurs) sonde pouvant être marquée, notamment par radioactivité ou par la technique des sondes froides, capable de s'hybrider spécifiquement avec la (ou les) séquence(s) d'acides nucléiques amplifiée(s) à détecter.

L'utilisation des amorces indiquées ci-dessus pour la mise en oeuvre d'un procédé de synthèse de protéines codées par les séquences nucléotidiques amplifiées à l'aide de ces amorces est aussi divulguée.

A titre illustratif, ce procédé de synthèse de protéines comprend l'amplification de séquences nucléotidiques des génomes des virus du type HIV ou SIV (codant pour une protéine déterminée et ayant subi, le cas échant, certaines modifications de leurs nucléotides) par mise en contact desdites séquences avec au moins un couple d'amorces selon l'invention dans les conditions décrites ci-dessus, suivie de la traduction de ces séquences ainsi amplifiées en protéines ; cette dernière étape est réalisée notamment par transformation de cellules hôtes appropiées à l'aide de vecteurs contenant lesdites séquences amplifiées, et récupération des protéines produites dans ces cellules hôtes.

Les polypeptides issus de la traduction des séquences (ou amorces) nucléotidiques de l'invention sont également concernés.

L'utilisation des amorces oligonucléotidiques anti-sens en tant qu'agents antiviraux en général, notamment dans la lutte contre le SIDA, ainsi que des compositions pharmaceutiques contenant ces amorces anti-sens en association avec un véhicule pharmaceutiquement acceptable, est également divulguée.

Sont décrites également les compositions immunogènes comprenant un ou plusieurs produits de traduction des séquences nucléotidiques divulguées et/ou un ou plusieurs produits de traduction des séquences nucléotidiques amplifiées selon les procédés décrits ci-dessus à partir des amorces définies selon l'invention, ces produits de traduction étant associés à un véhicule pharmaceutiquement acceptable.

Sont décrits les anticorps dirigés contre l'un ou plusieurs des produits de traduction décrits ci-dessus (ou en d'autres termes, susceptibles de former une réaction immunologique avec un ou plusieurs produits de traduction des séquences nucléotidiques selon l'invention, ou encore un ou plusieurs produits de traduction des séquences nucléotidiques amplifiées à partir des amorces définies selon la demande et leur utilisation pour la mise en oeuvre de méthodes de diagnostic in vitro de l'infection d'un individu par un virus du type HIV-1 et/ou HIV-2, ou d'un animal par au moins l'un des trois virus (HIV-1, HIV-2, SIV) selon les procédés connus de l'homme de l'Art.

A titre illustratif, une telle méthode de diagnostic in vitro comprend la mise en contact d'un échantillon biologique (notamment de sérum) prélevé, chez un patient à l'étude, avec des anticorps décrits et la détection à l'aide de tout procédé approprié (notamment à l'aide d'anti-immunoglobulines marquées) des complexes formés entre les antigènes des virus du type HIV ou SIV éventuellement présents dans l'échantillon biologique et lesdits anticorps.

Sont également décrits des kits de diagnostic in vitro comprenant des anticorps décrits et, le cas échéant, des réactifs appropriés à la mise en évidence de la réaction immunologique formée entre lesdits anticorps et les antigènes des virus HIV ou SIV.

Est visé également un procédé de préparation des polypeptides mentionnés ci-dessus, notamment ceux correspondant selon le code génétique universel aux séquences (ou amorces) nculéotidiques décrites ci-dessus, ce procédé étant caractérisé en ce que, partant de préférence de l'aminoacide C-terminal, l'on condense successivement deux à deux les aminoacyles successifs dans l'ordre requis, ou des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragment à l'exception des fonctions amine de l'un et carboxyle de l'autre ou vice-versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

Par exemple, on aura recours à la technique de synthèse peptidique en solution homogène décrite par Houbenweyl dans "Meuthode der Organischen Chemie" (Méthode de la chimie Organique) édité par E. Wunsch, vol. 15-I et II, THIEME, STUTTGART, 1974, ou à celle de synthèse peptidique en phase solide décrite par R.D. Merrifield dans "Solid Phase Peptide Synthesis" (J. AM. CHEM. SOC., 45, 2149-2154).

Est concerné également un procédé de préparation des séquences (ou amorces) nucléotidiques décrites ci-dessus, ce procédé comprenant les étapes suivantes :
- incubation de l'ADN génomique, isolé à partir d'un des virus du type Bis ou SIV sus-mentionnés, avec de l'ADNase 1, puis addition d'EDTA et purification par extraction au mélange phenol/chloroforme/alcool isoamylique (25/24/ 1) puis par l'éther,
- traitement de l'ADN ainsi extrait par de l'Eco R1 méthylase en présence de DTT, et purification par extraction telle que décrite ci-dessus,
- incubation de l'ADN ainsi purifié avec les 4 désoxynucléotides triphosphates dATP, dCTP, dGTP, et dTTP en présence de T4 ADN polymérase et d'ADN ligase de E. coli, puis purification selon la méthode décrite ci-dessus,
- le clonage des acides nucléiques ainsi obtenus dans un vecteur approprié et la récupération de l'acide nucléique recherché à l'aide d'une sonde appropriée.

Un procédé de préparation particulièrement avantageux des séquences nucléotidiques décrites comprend les étapes suivantes :
- la synthèse d'ADN en utilisant la méthode automatisée des β-cyanethyl phosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée.

Un autre procédé de préparation des séquences nucléotidiques décrites comprend les étapes suivantes :
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Natl. Acad. Sci. USA, 80; 7461-7465, (1983),
- le clonage des acides nucléiques ainsi obtenus dans un vecteur approprié et la récupération de l'acide nucléique recherché par hybridation avec une sonde appropriée.

## Revendications

1. Oligonucléotide **caractérisé en ce que** sa séquence consiste en :
i) une séquence spécifique du gène gag, et susceptible d'hybrider à une température de 60°C ± 1 °C avec les génomes des virus HIV-1 Bru, HIV-1 Mal, HIV-1 Eli, HIV-2 Rod et SIV Mac, ou
ii) une séquence complémentaire d'une séquence telle que définie en i.

2. Oligonucléotide selon la revendication 1, **caractérisé en ce qu'**il a une taille d'environ 15 à 30 nucléotides.

3. Oligonucléotide selon la revendication 1 **caractérisé en ce que** sa séquence nucléotidique a au moins 60% d'identité avec la séquence du gène *gag* des virus HIV-1 Bru, HIV-1 Mal, HIV-1 Eli, HIV-2 Rod ou SIV Mac, et garde les propriétés d'hybridation avec les génomes des virus HIV-1 Bru, HIV-1 Mal, HIV-1 Eli, HIV-2 Rod et SIV Mac.

4. Oligonucléotide selon la revendication 3, dont les 5 bases de chaque extrémité sont conservées par rapport à celles d'une amorce oligonucléotidique telle que décrite à l'une quelconque des revendications 1 à 3, et qui comporte dans sa partie médiane des modifications par rapport à la séquence d'une amorce oligonucléotidique selon l'une quelconque des revendications 1 à 3, et garde les propriétés d'hybridation des amorces oligonucléotidiques définies aux revendications 1 à 3, à une température de 60°C 1°C, avec les génomes des virus HIV-1 Bru, HIV-1 Mal, HIV-1 Eli, HIV-2 Rod et SIV Mac.

5. Oligonucléotide selon l'une quelconque des revendications 1 à 4, susceptible d'hybrider avec les génomes des virus HIV-1 Bru, HIV-1 Mal, HIV-1 Eli, HIV-2 Rod et SIV Mac dans un tampon de composition Tris-HCl, pH 8,9 : 50 mM ; (NH₄)₂ SO₄: 15 mM ; MgCl₂ : 5mM; β-mercapto-éthanol : 10 mM ; gélatine : 0,25 mg/ml.

6. Procédé d'amplification génique de séquences nucléiques du gène gag de virus du type HIV-1 et/ou HIV-2, et/ou SIV, réalisé à partir d'un échantillon biologique, ce procédé comprenant principalement les étapes suivantes:
a) une étape d'extraction de l'acide nucléique à détecter appartenant au génome du virus du type HIV-1, HIV-2, ou SIV éventuellement présent dans l'échantillon biologique susmentionné et, le cas échéant, une étape de traitement à l'aide d'une transcriptase inverse dudit acide nucléique si ce dernier est sous forme d'ARN,
**b)** un cycle comprenant les étapes suivantes :
- dénaturation de l'acide nucléique double brin à détecter, ce qui conduit à la formation d'un acide nucléique simple brin,
- hybridation de chacun des brins d'acide nucléique, obtenus lors de l'étape de dénaturation précédente, avec au moins une amorce selon l'une des revendications 1 à 5, par mise en contact des brins sus-mentionnés avec au moins un couple d'amorces sus-mentionnées,
- formation à partir des amorces des ADN complémentaires aux brins sur lesquels elles sont hybridées en présence d'une ADN polymérase et de quatre nucléosides triphosphate (dNTP) différents, ce qui conduit à la formation d'un plus grand nombre d'acides nucléiques double brin à détecter qu'à l'étape de dénaturation précédente,
ce cycle étant répété un nombre de fois déterminé pour obtenir ladite séquence nucléique à détecter éventuellement présente dans l'échantillon biologique dans une proportion suffisante pour permettre sa détection,
c) une étape de détection de la présence éventuelle de l'acide nucléique appartenant au génome du virus du type HIV-1 et/ou HIV-2 et/ou SIV dans l'échantillon biologique.

7. Procédé selon la revendication 6, **caractérisé en ce que** l'étape de dénaturation est réalisée en présence d'au moins une d'amorce selon l'une des revendications 1 à 5.

8. Procédé selon la revendication 7, **caractérisé en ce qu'**il est réalisé dans les conditions suivantes :
a) hybridation : les amorces (1 µl d'une solution à 40 µmolaire de chaque amorce) sont mis en présence de l'ADN-matrice (100 à 300 ng) pour la première étape de dénaturation-réassociation ; on chauffe, durant 10 minutes à 100 °C puis on plonge les tubes contenant ce mélange d'ADN-matrice et de primers dans de l'eau contenant de la glace. Les primers doivent être utilisés à une concentration finale dans l'étape d'amplification qui suit de 0,8 µM chaque,
b) amplification : on ajoute au milieu précédent les 4 dNTP chacun étant utilisé à 0,5 µmolaire en solution finale (50 µl), et une unité de Taq-polymérase pour un milieu réactionnel de 50 µl ;

9. Application du procédé selon l'une quelconque des revendications 6 à 8 au diagnostic in vitro de l'infection d'un individu par un virus du type HIV-1 et/ou HIV-2, ou d'un animal par au moins l'un des trois virus (HIV-1, HIV-2, SIV).

10. Kit pour la mise en oeuvre d'un procédé selon l'une des revendications 6 à 8 comprenant :
i) au moins une amorce oligonucléotidique selon l'une quelconque des revendications 1 à 5,
ii) des réactifs appropriés à la mise en oeuvre du cycle d'opérations d'amplification, notamment de l'ADN polymérase, quatre nucléotides triphosphate différents, et le tampon 10 x buffer tel que décrit dans la revendication 5,
iii) une (ou plusieurs) sonde, pouvant être marquée, capable de s'hybrider avec la (ou les) séquence d'acide nucléique amplifiée à détecter.

11. Utilisation d'au moins un oligonucléotide selon l'une quelconque des revendications 1 à 5 pour l'amplification génique de séquences nucléiques du gène gag de virus du type HIV-1 et/ou HIV-2, et/ou SIV.

## Claims

1. An oligonucleotide, **characterized in that** its sequence consists of:
i) a specific sequence of the gag gene, which is capable of hybridizing at a temperature of 60°C ± 1°C with genomes of the HIV-1 Bru, HIV-1 Mal, HIV-1 Eli, HIV-2 Rod and SIV Mac viruses; or
ii) a complementary sequence to a sequence defined in i).

2. An oligonucleotide according to claim 1, **characterized in that** it has a size of about 15 to 30 nucleotides.

3. An oligonucleotide according to claim 1, **characterized in that** its nucleotide sequence has at least 60% identity with the sequence of the gag gene of the HIV-1 Bru, HIV-1 Mal, HIV-1 Eli, HIV-2 Rod or SIV Mac viruses, and retains hybridization properties with the genomes of the HIV-1 Bru, HIV-1 Mal, HIV-1 Eli, HIV-2 Rod and SIV Mac viruses.

4. An oligonucleotide according to claim 3, for which the 5 bases of each end are conserved with respect to the sequence of an oligonucleotide primer as described in any one of claims 1 to 3, and which comprises, in its median portion, modifications with respect to the sequence of an oligonucleotide primer according to any one of claims 1 to 3, and retains the hybridization properties of the oligonucleotides primers as defined in any one of claims 1 to 3, at a temperature of 60°C ± 1°C with the genomes of the HIV-1 Bru, HIV-1 Mal, HIV-1 Eli, HIV-2 Rod and SIV Mac viruses:

5. An oligonucleotide according to any one of claims 1 to 4, which is capable of hybridizing with the genomes of the HIV-1 Bru, HIV-1 Mal, HIV-1 Eli, HIV-2 Rod and SIV Mac viruses in a buffer with the following composition: Tris-HCI, pH 8.9: 50 mM; (NH₄)₂SO₄: 15 mM; MgCl₂: 5 mM; β-mercapto-ethanol: 10 mM; gelatin: 0.25 mg/ml.

6. A method for genetic amplification of nucleic sequences of the gag gene of a HIV-1 and/or HIV-2 and/or SIV type virus, carried out from a biological sample, said method principally comprising the following steps:
a) a step for extracting nucleic acid to be detected belonging to the genome of the HIV-1, HIV-2 or SIV type virus which may be present in said biological sample and, if appropriate, a step for treating said nucleic acid using reverse transcriptase if the nucleic acid is in the form of RNA;
b) a cycle comprising the following steps:
• denaturing the double strand nucleic acid to be detected, resulting in the formation of a single strand nucleic acid;
• hybridizing each of the nucleic acid strands obtained during the preceding denaturing step with at least one primer in accordance with one of claims 1 to 5, by bringing said strands into contact with at least one pair of said primers;
• forming, starting from said primers, the complementary DNA to the strands on which they are hybridized in the presence of a DNA polymerase and four different nucleoside triphosphates (dNTP), resulting in the formation of a larger number of double strand nucleic acids to be detected than in the preceding denaturing step; said cycle being repeated a predetermined number of times to obtain said nucleic sequence to be detected which may be present in the biological sample in a proportion which is sufficient to allow its detection;
c) a step for detecting whether a nucleic acid belonging to the genome of the HIV-1 and/or HIV-2 and/or SIV type virus is present in the biological sample.

7. A method according to claim 6, **characterized in that** the denaturing step is carried out in the presence of at least one primer in accordance with one of claims 1 to 5.

8. A method according to claim 7, **characterized in that** it is carried out under the following conditions:
a) hybridization: bringing the primers (1 µl of a 40 µmolar solution of each primer) into the presence of matrix DNA (100 to 300 ng) for the first denaturing-reassociation step; heating for 10 minutes at 100°C then immersing the tubes containing said matrix DNA and primers mixture in water containing ice, the primers each being used in the subsequent amplification step at a final concentration of 0.8 µM;
b) amplification: adding the 4 dNTPs to the above medium, each being used at 0.5 µmolar in the final solution (50 µl), and one unit of Taq-polymerase for a reaction medium of 50 µl.

9. Application of the method according to any one of claims 6 to 8 to *in vitro* diagnosis of infection of an individual by a HIV-1 and/or HIV-2 type virus, or of an animal by at least one of the three viruses (HIV-1, HIV-2, SIV).

10. A kit for carrying out a method according to one of claims 6 to 8, comprising:
i) at least one oligonucleotide primer in accordance with any one of claims 1 to 5;
ii) reagents suitable for carrying out the cycle of amplification operations, in particular DNA polymerase, four different nucleoside triphosphates, and the 10 x buffer defined in claim 5;
iii) one (or more) probes, which may be labeled, capable of hybridizing with the amplified nucleic acid sequence or sequences to be detected.

11. Use of at least one oligonucleotide according to any one of claims 1 to 5 for gene amplification of nucleic sequences of the gag gene of a HIV-1 and/or HIV-2 and/or SIV type virus.

## Patentansprüche

1. Oligonukleotid, **dadurch gekennzeichnet, dass** dessen Sequenz besteht aus:
i) einer spezifischen Sequenz des Gens gag und das geeignet ist, bei einer Temperatur von 60°C ± 1°C mit den Genomen der Viren HIV-1 Bru, HIV-1 Mal, HIV-1 Eli, HIV-2 Rod und SIV Mac zu hybridisieren, oder
ii) einer Komplementärsequenz einer Sequenz, wie sie unter i) definiert ist.

2. Oligonukleotid gemäß Anspruch 1, **dadurch gekennzeichnet, dass** es eine Größe von ungefähr 15 bis 30 Nukleotiden hat.

3. Oligonukleotid gemäß Anspruch 1, **dadurch gekennzeichnet, dass** dessen Nukleotidsequenz mindestens 60% Identität mit der Sequenz des Gens gag der Viren HIV-Bru, HIV-1 Mal, HIV-1 Eli, HIV-2 Rod oder SIV Mac aufweist, und die Hybridisierungseigenschaften mit den Genomen der Viren HIV-1 Bru, HIV-1 Mal, HIV-1 Eli, HIV-2 Rod und SIV Mac behält.

4. Oligonukleotid gemäß Anspruch 3, dessen die 5 Basen an jedem Ende bezüglich der Basen eines Oligonukleotidprimers, wie er in einem der Ansprüchen 1 bis 3 beschrieben ist, konserviert sind, und das in dessen mittleren Teil Modifikationen bezüglich der Sequenz eines Oligonukleotidprimers gemäß einem der Ansprüchen 1 bis 3 aufweist, und das die Hybridisierungseigenschaften der Oligonukleotidprimer gemäß Ansprüchen 1 bis 3, bei einer Temperatur von 60°C ± 1°C mit den Genomen der Viren HIV-1 Bru, HIV-1 Mal, HIV-1 Eli, HIV-2 Rod und SIV Mac behält.

5. Oligonukleotid gemäß einem der Ansprüche 1 bis 4, das geeignet ist, mit den Genomen der Viren HIV-1 Bru, HIV-1 Mal, HIV-1 Eli, HIV-2 Rod und SIV Mac in einem Puffer der Zusammensetzung Tris-HCl, pH = 8,9: 50 mM; (NH₄)₂SO₄: 15 mM; MgCl₂: 5 mM; β-Mercaptoethanol: 10 mM; Gelatine: 0,25 mg/ml zu hybridisieren.

6. Verfahren der Genamplifikation von Nukleinsäuresequenzen des Gens gag von Virus vom Typ HIV-1 und/oder HIV-2 und/oder SIV, durchgeführt ausgehend von einer biologischen Probe, wobei dieses Verfahren hauptsächlich die folgenden Schritte umfasst:
a) einen Schritt der Extraktion der nachzuweisenden Nukleinsäure, die zum Genom von Virus vom Typ HIV-1, HIV-2 oder SIV gehört, der möglicherweise in der oben erwähnten biologischen Probe vorhanden ist, und gegebenenfalls einen Schritt der Behandlung dieser Nukleinsäure mit Hilfe einer reversen Transkriptase, wenn diese Nukleinsäure in Form von RNA vorliegt,
b) einen Zyklus, der folgende Schritte umfasst:
- Denaturierung der nachzuweisenden doppelsträngigen Nukleinsäure, was zur Bildung einer einzelsträngigen Nukleinsäure führt,
- Hybridisierung von jedem der Nukleinsäurestränge, die bei dem vorhergehenden Denaturierungsschritt erhalten wurden, mit mindestens einem Primer gemäß einem der Ansprüche 1 bis 5 durch Inkontaktbringen der oben erwähnten Stränge mit mindestens einem Paar der oben erwähnten Primer,
- Bildung, ausgehend von DNA-Primern, die zu den Strängen komplementär sind, auf die sie hybridisiert sind, in Gegenwart einer DNA-Polymerase und vier unterschiedlichen Nukleosidtriphosphaten (dNTP), was zur Bildung einer größeren Anzahl von nachzuweisenden doppelsträngigen Nukleinsäuren als beim vorhergehenden Schritt der Denaturierung führt,
wobei dieser Zyklus eine bestimmte Anzahl von Malen wiederholt wird, um die möglicherweise in der biologischen Probe vorhandene, nachzuweisende Nukleinsäuresequenz in einem Ausmaß zu erhalten, das ausreicht, deren Nachweis zu ermöglichen,
c) einen Schritt des Nachweises eines möglichen Vorhandenseins von Nukleinsäure, die zum Genom von Virus vom Typ HIV-1 und/oder HIV-2 und/oder SIV gehört, in der biologischen Probe.

7. Verfahren gemäß Anspruch 6, **dadurch gekennzeichnet, dass** der Denaturierungsschritt in Gegenwart von mindestens einem Primer gemäß einem der Ansprüche 1 bis 5 durchgeführt wird.

8. Verfahren gemäß Anspruch 7, **dadurch gekennzeichnet, dass** es unter folgenden Bedingungen durchgeführt wird:
a) Hybridisierung: Die Primer (1 µl einer Lösung zu 40 µmolar an jedem der Primer) werden für den ersten Schritt der Denaturierung-Reassoziierung mit DNA-Matrix (100 bis 300 ng) zusammengegeben; es wird 10 Minuten lang auf 100°C erhitzt, und daraufhin werden die Gefäße, die diese Mischung aus DNA-Matrix und Primern enthalten, in Eis-haltiges Wasser getaucht. Die Primer sollen im Amplifikationsschritt in einer Endkonzentration verwendet werden, die jeweils 0,8 µM beträgt.
b) Amplifikation: Dem vorher beschriebenen Medium werden die 4 dNTPs, wobei jedes zu 0,5 µmolar in der schließlich erhaltenen Lösung (50 µl) eingesetzt wird, und eine Einheit Taq-Polymerase für ein Reaktionsmedium von 50µl zugegeben.

9. Verwendung des Verfahrens gemäß einem der Ansprüche 6 bis 8 zur *in vitro-*Diagnose der Infektion eines Individuums mit einem Virus vom Typ HIV-1 und/oder HIV-2, oder eines Tieres mit mindestens einem der drei Viren (HIV-1, HIV-2, SIV).

10. Kit zur Durchführung eines Verfahrens gemäß einem der Ansprüche 6 bis 8, das umfasst:
i) mindestens einen Oligonukleotidprimer gemäß einem der Ansprüche 1 bis 5,
ii) geeignete Reagenzien zur Durchführung des Zyklus der Arbeitsgänge der Amplifikation, insbesondere DNA-Polymerase, vier unterschiedliche Nukleotidtriphosphate und den Puffer "10 x buffer", der in Anspruch 5 beschrieben ist,
iii) eine (oder mehrere) Sonde(n), die markiert sein kann (können) und die in der Lage ist (sind), mit der (den) amplifizierten nachzuweisenden Nukleinsäuresequenz(en) zu hybridisieren.

11. Verwendung von mindestens einem Oligonukleotid gemäß einem der Ansprüche 1 bis 5 zur Genamplifikation von Nukleinsäuresequenzen des Gens *gag* von Virus vom Typ HIV-1 und/oder HIV-2 und/oder SIV.
